# EUROPEAN PATENT APPLICATION

(11) **EP 4 410 305 A1**
(43) Date of publication of application: **07.08.2024**
(21) Application number: 22876850.3
(22) Date of filing: 28.09.2022
(51) Int. Cl.: A61K 39/00, A61P 35/00

(54) **ANTICANCER VACCINE COMPOSITION COMPRISING HSP90 ANTIGENIC PEPTIDE AND USE THEREOF**

(30) Priority: 29.09.2021 KR 20210128516
(71) Applicant: Aston Sci. Inc., Seoul 06193 (KR); Cha Vaccine Research Institute Co., Ltd, Seongnam-si, Gyeonggi-do 13230 (KR)
(72) Inventor: KANG, Jin Ho, Seoul 02855 (KR); PARK, Hyo Hyeon, Seoul 05229 (KR); JUNG, Hun, Seoul 05572 (KR); YUM, Jung Sun, Seongnam-si, Gyeonggi-do 13230 (KR); AHN, Byung Cheol, Seongnam-si, Gyeonggi-do 13230 (KR); JUNG, Eun Jung, Seongnam-si, Gyeonggi-do 13230 (KR)
(74) Representative: ZBM Patents - Zea, Barlocci & Markvardsen
(86) International application number: PCT/KR2022/014566
(87) International publication number: WO 2023/055078

(57) **Abstract**

The present disclosure relates to an anticancer vaccine composition including a peptide of SEQ ID NO: 1 and a peptide of SEQ ID NO: 2, which are epitopes of HSP90, and the vaccine composition according to the present disclosure may effectively inhibit the growth of tumors in an animal model of tumor cell line transplantation without severe adverse effects, and thus may be useful for treating cancer or preventing cancer recurrence.

## Description

### Technical Field

The present application claims the benefit of priority to Korean Patent Application No. 10-2021-0128516, filed September 29, 2021, and all of the disclosure in that Korean patent application is hereby incorporated by reference as part of this specification.

The present disclosure relates to an anticancer vaccine composition including an HSP90 antigenic peptide for cancer treatment and recurrence prevention and use thereof.

### Background Art

Currently, cancer is one of biggest diseases in humans, along with heart disease, and is the number one cause of death among Koreans, but the cause is not yet clearly known. Various treatment methods such as surgical therapy, radiation therapy, and chemotherapy have been designed to treat cancer, and each treatment method is applied singly or in combination depending on the type of cancer, but there are patients who are not completely cured due to acquisition of resistance to anticancer agents, and in some cases, treatment is difficult due to high metastasis and recurrence rates.

Vaccines help the body fight disease by training the immune system to recognize and destroy harmful substances and diseased cells. Vaccines are broadly categorized into two types, prophylactic vaccines and therapeutic vaccines. A prophylactic vaccine is a form of traditional vaccine, provided to healthy people to prevent the development of a particular disease, while a therapeutic vaccine, also called immunotherapy, is provided to a person diagnosed with a disease as a method to inhibit or prevent the growth and development of the disease. A typical form of a therapeutic vaccine is an anticancer vaccine (also known as a cancer vaccine).

Anticancer vaccines are designed to activate cytotoxic T cells, so that the activated T cells recognize and act against specific types of cancer cells, or induce the production of antibodies that bind to antigens present on the surface of cancer cells. The basic therapeutic goal of anticancer vaccines is to reduce the size of cancer tissue already present in a cancer patient or to inhibit recurrence after removing the cancer tissue of the cancer patient through surgery or chemotherapy.

Over the past few years, extensive vaccine research has been conducted to treat cancer or prevent recurrence, but with the exception of the prostate cancer vaccine sipuleucel-T (Provenge^{®}), there are no successful cases yet. This low success rate has been attributed in part to the fact that although the antigen is specifically expressed on a certain type of tumor cell, it may be expressed at a low level depending on each individual, or in the case of a metastatic tumor, the antigenic profile may change between the first tumor and metastatic tumor, and immune evasion mechanisms in tumor cells.

Desirable vaccine antigens should be expressed exclusively or at least at increased levels on tumor cells. Heat Shock Protein 90 (HSP90) is known to be overexpressed in many carcinomas, and it is known that vaccination with a specific antigenic region (epitope) of HSP90 shows an antitumor effect in animal models.

An adjuvant is an ingredient included together with an antigen in a vaccine composition and has been developed for the purpose of controlling or enhancing the immune response responding to the antigen. The adjuvant is not immunogenic in itself when administered alone, but when co-administered with an antigen, it may enhance and sustain an immune response to the antigen. Typical examples of an adjuvant include aluminum-based inorganic matters such as potassium alum and aluminum hydroxide, etc., oils such as paraffin oil, etc., saponin surfactant, and Freund's complete adjuvant/Freund's incomplete adjuvant (CFA/IFA).

Recently, as it has become known that the innate immune system is activated when a toll-like receptor (TLR) is activated, there have been attempts to use TLR agonists that activate the TLR as adjuvants. TLR is a transmembrane receptor protein and is known to be mainly expressed in innate immune cells. As a subtype of TLR, TLR1, TLR2, TRL4, TLR5, and TLR6 are known to be expressed on the cell surface, and TLR3, TLR7, TLR8, and TLR9 are known to be expressed on the endosomal membrane of an intracellular endosome.

Recently, research has been attempted to derive an optimized combination of antigen and aluminum-based adjuvant to produce a vaccine that exhibits an optimized immune response. However, for cancer vaccines using HSP90 antigenic peptides, no optimized adjuvant has been identified.

### [Prior Art Documents]

### [Patent Documents]

(Patent Document 1) KR 10-1501583 (Publication date: March 12, 2015)
(Patent Document 2) KR 10-2098097 (Publication date: May 26, 2020)
(Patent Document 3) KR 10-2256267 (Publication date: May 26, 2021)

### [Non-patent Documents]

(Non-patent Document 1) npj Vaccines 3, Article number: 51 (October 10, 2018)
(Non-patent Document 2) Pharmaceutics 13, 142 (January 21, 2021)

### Disclosure

### Technical Problem

Accordingly, the inventors of the present disclosure conducted research to solve the above problem and completed the present disclosure by confirming that an anticancer vaccine composition including an HSP90 antigenic peptide and a TLR agonist as adjuvants may effectively inhibit tumor growth in a tumor mouse model into which cancer cells were transplanted.

The objective of the present disclosure is to provide an anticancer vaccine composition including: one or more peptides selected from the group consisting of a peptide including an amino acid sequence of SEQ ID NO: 1 and a peptide including an amino acid sequence of SEQ ID NO: 2; and a TLR2 agonist and a TLR3 agonist as an adjuvant.

The TLR2 agonist may be one or more selected from Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, and Dhc-SKKKK.

The TLR2 agonist may be Pam3Cys-SKKKK.

The TLR3 agonist may be a polyinosinic:polycytidylic acid (poly (I:C)).

The poly (I:C) may have a length of 50 bp to 2,000 bp.

The anticancer vaccine composition may be for treating cancer and/or preventing cancer recurrence in a subject.

The subject may be a breast cancer patient.

The anticancer vaccine composition may not include a liposome.

The anticancer vaccine composition may additionally include one or more agents selected from an immunomodulator, a chemical anticancer agent, a targeted anticancer agent, and an immune anticancer agent.

The immune anticancer agent may be an immune checkpoint inhibitor against one or more selected from the group consisting of CTLA-4, PD-1, LAG-3, TIM-3, TIGIT, and VISTA.

The objective of the present disclosure is to provide an anticancer vaccine composition including: one or more nucleic acids selected from the group consisting of a nucleic acid including a sequence encoding a peptide including the amino acid sequence of SEQ ID NO: 1 and a nucleic acid including a encoding sequence of a peptide including the amino acid sequence of SEQ ID NO: 2; and a TLR2 agonist and a TLR3 agonist as an adjuvant.

The TLR2 agonist may be one or more selected from Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, and Dhc-SKKKK.

The TLR2 agonist may be Pam3Cys-SKKKK.

The TLR3 agonist may be a polyinosinic:polycytidylic acid (poly (I:C)).

The poly (I:C) may have a length of 50 bp to 2,000 bp.

The anticancer vaccine composition may be for treating cancer and/or preventing cancer recurrence in a subject.

The subject may be a breast cancer patient.

The anticancer vaccine composition may not include a liposome.

The anticancer vaccine composition may additionally include one or more agents selected from an immunomodulator, a chemical anticancer agent, a targeted anticancer agent, and an immune anticancer agent.

The immune anticancer agent may be an immune checkpoint inhibitor against one or more selected from the group consisting of CTLA-4, PD-1, LAG-3, TIM-3, TIGIT, and VISTA.

The objective of the present disclosure is to provide a pharmaceutical composition for the treatment or prevention of cancer including the anticancer vaccine composition.

The objective of the present disclosure is to provide a kit for the treatment of cancer including the anticancer vaccine composition.

The objective of the present disclosure is to provide a method of inhibiting cancer progression and/or recurrence by administering the anticancer vaccine composition, pharmaceutical composition or kit for cancer treatment or prevention to a subject.

The objective of the present disclosure is to provide a method of preventing, ameliorating or treating cancer, the method including administering the anticancer vaccine composition or a pharmaceutical composition or kit for treating or preventing cancer to a subject.

The objective of the present disclosure is to provide use of the anticancer vaccine composition, or a pharmaceutical composition or kit for treating or preventing cancer, for the manufacture of a medicament for prevention, amelioration or treatment of cancer.

### Technical Solution

To accomplish the above objective, the present disclosure provides an anticancer vaccine composition including: one or more peptide selected from the group consisting of a peptide including the amino acid sequence of SEQ ID NO: 1 and a peptide including the amino acid sequence of SEQ ID NO: 2 as an HSP90 antigenic peptide; and a TLR2 agonist and a TLR3 agonist as an adjuvant.

In addition, the present disclosure provides an anticancer vaccine composition including: one or more nucleic acid selected from the group consisting of a nucleic acid including a sequence encoding a peptide including an amino acid sequence of SEQ ID NO: 1 and a nucleic acid including a sequence encoding a peptide including an amino acid sequence of SEQ ID NO: 2 as a nucleic acid including a sequence encoding an HSP90 antigenic peptide; and a TLR2 agonist and a TLR3 agonist as an adjuvant.

In addition, the present disclosure provides a method of preventing, ameliorating, or treating cancer, the method including administering to a subject: one or more peptides selected from the group consisting of a peptide including the amino acid sequence of SEQ ID NO: 1 and a peptide including the amino acid sequence of SEQ ID NO: 2; and a TLR2 agonist and a TLR3 agonist as an adjuvant.

Additionally, the present disclosure provides use of a peptide and an adjuvant for the manufacture of a medicament for prevention, amelioration, or treatment of cancer, wherein the peptide may be at one or more selected from the group consisting of a peptide including the amino acid sequence of SEQ ID NO: 1 and a peptide including the amino acid sequence of SEQ ID NO: 2, and the adjuvant may include a TLR2 agonist and a TLR3 agonist.

According to an embodiment of the present disclosure, the anticancer vaccine composition may include a peptide including the amino acid sequence of SEQ ID NO: 1 and a peptide including the amino acid sequence of SEQ ID NO: 2 as the HSP90 antigenic peptide; and a TLR2 agonist and TLR3 agonist as an adjuvant.

According to an embodiment of the present disclosure, the TLR2 agonist may be one or more selected from Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, and Dhc-SKKKK.

According to an embodiment of the present disclosure, the TLR3 agonist may be a polyinosinic:polycytidylic acid (poly (I:C)).

According to an embodiment of the present disclosure, the anticancer vaccine composition may not include a liposome.

According to an embodiment of the present disclosure, the anticancer vaccine composition may be used to treat cancer or prevent recurrence in a subject.

According to an embodiment of the present disclosure, the cancer may be a solid tumor.

According to an embodiment of the present disclosure, the cancer may be breast cancer.

According to an embodiment of the present disclosure, the cancer patient group may be a solid tumor patient group.

According to an embodiment of the present disclosure, the group of cancer patients may be a group of breast cancer patients.

According to an embodiment of the present disclosure, provided is a method of treating or preventing a cancer patient using (administering) an anticancer vaccine composition.

### Advantageous Effects

The vaccine composition according to the present disclosure includes HSP90 antigenic peptide; and a TLR2 agonist and a TLR3 agonist as an adjuvant, the vaccine composition shows higher immunogenicity than when other types of TLR agonists, such as a TLR4 agonist, etc. is used as an adjuvant, and may effectively inhibit tumor growth in a tumor mouse model transplanted with cancer cells, which may be useful for cancer treatment and prevention of recurrence.

### Description of Drawings

FIG. 1 shows the results of analyzing the Th1 immune response of a vaccine composition including HSP 90 antigenic peptide and various types of adjuvants.
FIG. 2 shows the results of analyzing the Th2 immune response of a vaccine composition including HSP 90 antigenic peptide and various types of adjuvants.
FIG. 3 shows the results of analyzing the immunogenicity of a vaccine composition including HSP 90 antigenic peptide and various subtypes of TLR agonists as adjuvants.
FIG. 4 shows the results of analyzing the anticancer effect of a vaccine composition including HSP 90 antigenic peptide and TLR 2/3 agonists as adjuvants.
FIG. 5 illustrate the actual tumor size and spleen size when a vaccine composition including HSP 90 antigenic peptide and TLR 2/3 agonist as an adjuvant administered to a tumor model mouse.

### Best Mode

Hereinafter, the present disclosure will be described in more detail.

In describing and claiming the present disclosure, the following terms will be used according to the definitions set forth below unless otherwise specified.

As used herein, when a part "includes" a certain component, this means that it may further include other components rather than excluding other components, unless specifically stated to the contrary.

It should be understood that although certain aspects are described herein with the term "including", other similar aspects described in terms of "consisting of" and/or "consisting essentially of" are also provided.

The terms "treat" and "anticancer effect" refer to any action that ameliorate or beneficially change the symptom of a cancer disease by administering the composition of the present disclosure, and specifically, when a cancer patient is inoculated with the anticancer vaccine composition of the present disclosure and the patient is examined by radiation or a physical method, it not only refers to the results or signs of results that prevent or reduce tumor growth, as well as after surgery or administration of anticancer agents to a cancer patient to remove cancerous tissue, it refers to the result or sign of the result of preventing cancer recurrence/relapse when the patient is inoculated with the vaccine composition of the present disclosure and then examined by radiation or physical methods.

The term "patient" or "subject" refers to a condition in which a disease may be prevented or treated by administration of the vaccine composition of the present disclosure, for example a living organism suffering from, treated for, or susceptible to cancer, specifically a solid tumor, and includes both humans and animals. The subject includes, but is not limited to, a mammal (for example, mouse, monkey, horse, cow, pig, dog, cat, etc.) and is preferably a human. In addition, "patient" or "subject" as used herein includes, without distinction, cancer patients, solid tumor patients, breast cancer patients, or breast cancer patients expressing HER2.

The term "administering" refers to introducing a vaccine composition into a subject using any of a variety of methods and delivery systems known in the art to which this invention belongs. Example routes of administration include intravenous, intramuscular, subcutaneous, intraperitoneal, spinal, or other parenteral routes of administration, for example by injection or infusion. "Administration" as used herein generally refers to subcutaneous or intradermal administration by injection, and includes, but is not limited to, Intravenous, intramuscular, intra-arterial, intrathecal, or intralymphatic injections and infusions. Other parenteral routes include topical, epithelial, or mucosal routes of administration, for example intranasal, vaginal, rectal, or sublingual route. In the present disclosure, "vaccine injection", "treatment", and "vaccination", which are the act of administering a vaccine to a subject, are used without distinction.

The term "antigenic peptide" refers to a set of amino acid residues in an antigen-binding site recognized by a specific antibody, or an epitope recognized by a T cell receptor (TCR) and/or major histocompatibility complex (MHC) receptor on a T cell.

In the present disclosure, the term "immune anticancer agent" refers to a therapeutic agent that induces immune cells to selectively attack only cancer cells by injecting artificial immune proteins into the body to stimulate the immune system, unlike existing anticancer agents that attack the cancer itself, which is an anticancer agent that restores or strengthens the tumor recognition or destruction ability of the immune system to overcome the immunosuppression or immune evasion mechanism acquired by cancer cells. The immune anticancer agent include, but are not limited to, an immune checkpoint inhibitor, an immune cell therapeutic, and an immunoviral therapeutic.

In the present disclosure, the term "immune checkpoint inhibitor" is a type of immuno-anticancer agent that acts to attack cancer cells by activating T cells by blocking the activation of Immune Checkpoint Protein, which is involved in inhibiting T cells, when some cancer cells evade immunity by utilizing the immune checkpoint of T cells, which are immune cells in the body, Including, a CTLA-4 inhibitor, PD-1 inhibitor, LAG-3 inhibitor. TIM-3 inhibitor, TIGIT inhibitor, and VISTA inhibitor, but is not limited thereto. Furthermore, the immune checkpoint inhibitor may be an antagonist, which may be an antagonist antibody or a small molecule compound.

Heat shock protein 90 (HSP90) is a type of chaperone protein that acts for protein folding, maturation, and conformational stabilization to form an intact structure of all peptide chains synthesized within cells. The expression level of HSP90 protein or gene is about 2 to 10 times higher in malignant cells than in normal cells, and HSP90 is predicted to play a major role in the survival and growth of cancer cells and is emerging as an effective drug target.

In addition, previous studies have reported that the concentration of auto-antibodies (anti-HSP antibodies) against HSP90 is higher in the serum of breast cancer patient group than in the control group (normal individual + benign breast cancer patient) (Lancet, 1995: 345, 126).

The anticancer vaccine composition of the present disclosure may include: one or more peptides selected from the group consisting of a peptide including an amino acid sequence of SEQ ID NO: 1 (first peptide) and a peptide including an amino acid sequence of SEQ ID NO: 2 (second peptide); and a TLR2 agonist and a TLR3 agonist as an adjuvant.

According to an embodiment, the anticancer vaccine composition may include a peptide including an amino acid sequence of SEQ ID NO: 1 (first peptide) and a peptide including an amino acid sequence of SEQ ID NO: 2 (second peptide); and a TLR2 agonist and a TLR3 agonist as an adjuvant.

The first peptide may be a peptide including the amino acid sequence of SEQ ID NO: 1, and specifically may be a peptide consisting of the amino acid sequence of SEQ ID NO: 1. In addition, the second peptide may be a peptide including the amino acid sequence of SEQ ID NO: 2, and specifically may be a peptide consisting of the amino acid sequence of SEQ ID NO: 2.

The HSP 90 antigenic peptide of the peptide represented by the amino acid sequence of SEQ ID NO: 1 and the peptide represented by the amino acid sequence of SEQ ID NO: 2 of the present disclosure was selected from among 12 15-mer peptide sequences expected to have good binding affinity to the MHC class II allele using five algorithm programs for the HSP90 full sequence (Korean Registered Patent No. 10-2256267).

The terms "peptide represented by the amino acid sequence of SEQ ID NO: X," "peptide consisting of the amino acid sequence of SEQ ID NO: X," and "peptide of SEQ ID NO: X" are used interchangeably and without distinction in the description of the present disclosure.

In some embodiments of the present disclosure, the peptide represented by the amino acid sequence of SEQ ID NO: 1 (LYVRRVFIMDNCEEL) and the peptide represented by the amino acid sequence of SEQ ID NO: 2 (PyroGlu-SKILKVIRKNLVKK) may each be chemically synthesized and used.

The vaccine composition of the present disclosure may include a peptide including the amino acid sequence of SEQ ID NO: 1 (first peptide) and a peptide including the amino acid sequence of SEQ ID NO: 2 (second peptide), each included in the form of a separate isolated peptide or in a form that is directly or indirectly linked to each other. When the first peptide and the second peptide are included in the form of being directly or indirectly connected to each other, the second peptide may be linked to the N-terminus or C-terminus of the first peptide directly or indirectly through a linker. The linker is not particularly limited as long as the peptide exhibit anticancer vaccine activity, and may be composed of, for example, 1 to 50 amino acids.

The vaccine compositions disclosed herein may be prepared as a peptide vaccine, a DNA vaccine, or an mRNA vaccine.

In an embodiment of the present disclosure, the anticancer vaccine composition may include: one or more nucleic acids selected from the group consisting of a nucleic acid including an encoding sequence of a peptide including an amino acid sequence of SEQ ID NO: 1 (first peptide) and a nucleic acid including an encoding sequence of a peptide including an amino acid sequence of SEQ ID NO: 2 (second peptide); and a TLR2 agonist and a TLR3 agonist, as an adjuvant.

According to an embodiment, the anticancer vaccine composition may include a nucleic acid including an encoding sequence of a peptide including an amino acid sequence of SEQ ID NO: 1 (first peptide) and a nucleic acid including an encoding sequence of a peptide including an amino acid sequence of SEQ ID NO: 2 (second peptide); and a TLR2 agonist and a TLR3 agonist as an adjuvant.

The nucleic acid including the encoding sequence of the first peptide may be named a first nucleic acid, and the first nucleic acid may be a nucleic acid including the encoding sequence of the first peptide, and specifically a nucleic acid consisting of the encoding sequence of the first peptide. In addition, the nucleic acid including the encoding sequence of the second peptide may be named a second nucleic acid, and the second nucleic acid may be a nucleic acid including the encoding sequence of the second peptide, and specifically a nucleic acid consisting of the encoding sequence of the second peptide.

The nucleic acid may be DNA or RNA (mRNA).

In some embodiments of the present disclosure, when the anticancer vaccine composition is prepared in a form of a DNA vaccine, the DNA vaccine may include for example, a promoter, an appropriate transcriptional and translational control element and one or more DNA molecules (for example, plasmid) having one or more of a nucleic acid sequence including the encoding sequence of a peptide including the amino acid sequence of SEQ ID NO: 1 (first nucleic acid), and a nucleic acid sequence including the encoding sequence of a peptide including the amino acid sequence of SEQ ID NO: 2 (second nucleic acid) of the present disclosure, and the plasmid may additionally include an enhance sequence, for example, a sequence that enhance expression level, intracellular targeting, etc.

Additionally, in the DNA vaccine, the first nucleic acid and the second nucleic acid may each be included in a separate plasmid, or may be directly or indirectly linked to each other and included in a single plasmid. When the first nucleic acid and the second nucleic acid are linked to each other and included in one plasmid, the second nucleic acid may be linked to the 5'-end or 3'-end of the first nucleic acid directly or indirectly through a linker. The linker is not particularly limited as long as the peptide encoded by the nucleic acid exhibits anticancer vaccine activity, and may be, for example, composed of 1 to 150 nucleotides. Furthermore, when the first nucleic acid and the second nucleic acid are included in a single plasmid, the first peptide and the second peptide translated therefrom may be translated as separate and distinct peptides, or the first peptide and the second peptide may be translated in a form in which the first peptide and the second peptide are directly or indirectly linked to each other.

In some embodiments of the present disclosure, when the anticancer vaccine composition is prepared in the form of an mRNA vaccine, for example, the mRNA vaccine may include one or more mRNA molecules having one or more of a nucleic acid sequence including a coding sequence of a peptide including the amino acid sequence of SEQ ID NO: 1 (first nucleic acid) of the present disclosure and a nucleic acid sequence including a coding sequence of a peptide including the amino acid sequence of SEQ ID NO: 2 (second nucleic acid), wherein the mRNA molecule may additionally include one or more selected from the group consisting of a 5'-cap, a 5'-untranslated region (UTR), a 3'-untranslated region, and a poly(A) tail.

Additionally, in the mRNA vaccine, the first nucleic acid and the second nucleic acid may each be included in a separate mRNA molecule, or may be directly or indirectly linked to each other and included in a single mRNA molecule. When the first nucleic acid and the second nucleic acid are linked to each other and included in one mRNA molecule, the second nucleic acid may be linked to the 5'-end or 3'-end of the first nucleic acid directly or indirectly through a linker. The linker is not particularly limited as long as the peptide encoded by the nucleic acid exhibits anticancer vaccine activity, and may be, for example, composed of 1 to 150 nucleotides. Furthermore, when the first nucleic acid and the second nucleic acid are included in a single mRNA molecule, the first peptide and the second peptide translated therefrom may be translated as separate and distinct peptides, or the first peptide and the second peptide may be translated in a form in which the first peptide and the second peptide are directly or indirectly linked to each other.

When the vaccine composition of the present disclosure is a DNA vaccine or an mRNA vaccine, the vaccine may consist of a nucleic acid including A (Adenine), T (Thymine), G (Guanine), C (Cytosine), U (Uridine) and/or modified forms thereof. For example, the modified forms may include one or more selected from the group consisting of 5-methylcytidine (5mC), N6-methyladenosine (m6A), 3,2'-O-dimethyluridine (m4U), 2-thiouridine (s2U), 2' fluorouridine, pseudouridine, 2'-O-methyluridine (Um), 2' deoxyuridine (2' dU), 4-thiouridine (s4U), 5-methyluridine (m5U), 2'-O-methyladenosine (m6A), N6, 2'-O-dimethyladenosine (m6Am), N6,N6,2'-O-trimethyladenosine (m62Am), 2'-O-methylcytidine (Cm), 7-methylguanosine (m7G), 2'-O-methylguanosine (Gm), N2,7-dimethylguanosine (m-2,7G), N2,N2,7-trimethylguanosine (m-2,2,7G), and N1-methyl-pseudouridine.

The vaccine composition of the present disclosure includes the antigenic peptides described above (peptides including the amino acid sequences of SEQ ID NO: 1 and SEQ ID NO: 2), as well as an adjuvant including a TLR2 agonist and a TLR3 agonist (Korean Registered Patent No. 10-1501583).

An adjuvant is any compound or substance that, generally when combined with a vaccine antigen, increases the immune response to the vaccine antigen relative to the immune response induced by the antigen alone. The adjuvant functions to increase the level of humoral immune response, cellular immune response, or both immune responses, and additionally has the function of sustaining these responses.

Commonly used adjuvants include aluminum-based inorganic matters such as potassium alum and aluminum hydroxide, etc., oils such as paraffin oil, etc., saponin surfactant, and Freund's complete adjuvant/Freund's incomplete adjuvant, etc., and a TLR agonist, which are ligands that activate Toll-like receptors (TLR), are currently being studied.

A TLR2 agonist is a lipopeptide synthetic analog derived from bacteria and mycoplasma, and various lipopeptide analogs such as am3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, Dhc-SKKKK have been synthesized. Furthermore, the fatty acids and amino acids of the above lipopeptides may be chemically modified. In the present disclosure, "TLR2 agonist" and "TLR2L" are used interchangeably without distinction.

In an embodiment of the present disclosure, the vaccine composition includes Pam3Cys-SKKKK as the TLR2 agonist, but is not limited thereto.

The TLR3 agonist, poly (I:C) (polyinosinic-polycytidylic acid), is a synthetic analog of double-stranded RNA and is known to induce Th1 immune responses in in vitro or in vivo studies. Moreover, poly (I:C) is known to stably mature dendritic cells (DC), which are antigen presenting cells (APC). In the present disclosure, "TLR3 agonist" and "TLR3L" are used interchangeably without distinction.

In an embodiment of the present disclosure, the vaccine composition includes poly (I:C) with a length of 50 bp to 2,000 bp as the TLR3 agonist, but is not limited thereto.

In a previous study, it was reported that an adjuvant mixed with a lipopeptide-derived TLR2 agonist and liposome followed by mixing a TLR3 agonist enhances the Th1 immune response (Korean Registered Patent No. 10-2098097)..

The liposome has a monolayer or multilayer lipid-bilayer membrane structure that is most similar to a cell membrane in the form of a phospholipid bilayer matrix. The lipid may be a cationic, anionic or neutral lipid. For example, the lipids include 1,2-dioleoyl-3-trimethylammonium-propane (DOTAP), 1,2-dioleoyl-sn-glycero-3-phosphoethanolamine (DOPE), dimethyldioctadecylammonium (DDA), DC-chol(3β-[N-(N',N'-Dimethylaminoethane)- carbamoyl]cholesterol), 1,2-dioleoyl-sn-glycero-3-[phospho-rac-(1-glycerol)] (DOPG), 1,2- dipalmitoyl-sn-glycero-3-phosphocholine (DPPC), 1,2-dioleoyl-sn-glycero-3-phosphocholine (DOPC), and/or cholesterol.

The liposome is effective in delivering both hydrophilic and hydrophobic substances due to the structural characteristics, and have the advantage of being able to be used selectively. Therefore, since the drug is slowly released during slow decomposition, it is used as a drug carrier for vaccines and has the advantage of reducing the amount of drug, which is one of the essential considerations when manufacturing a vaccine composition.

However, the vaccine composition of the present disclosure not only confirmed that including both TLR2 agonist and TLR3 agonist as an adjuvant to the aforementioned HSP90 antigenic peptide resulted in a higher level of Th1 immune response than including the TLR2 agonist (TLR2L) or TLR3 agonist (TLR3L) alone, but surprisingly, it was confirmed that the increased Th1 immune response was almost eliminated when further liposome was included as an adjuvant.

Therefore, although it has been known that when a TLR2/3 agonist (TLR2 agonist and TLR3 agonist) and liposomes are mixed and used as an adjuvant, the immune effect is increased compared to the TLR2/3 agonist, it may be seen that in the vaccine composition of the present disclosure, the liposome play a negative role rather than a synergic effect.

Considering the above results, it is preferable that the vaccine composition of an embodiment of the present disclosure does not include a liposome.

In the present disclosure, "TLR2 agonist and TLR3 agonist" and "TLR2/3L" are used interchangeably without distinction.

According to an embodiment, the HSP90 antigenic peptides of the present disclosure, the peptides of SEQ ID NO: 1 and SEQ ID NO: 2, barely induces a Th1 immune response in the case of using other types of TLR agonists, such as TLR4 agonist (TLR4) or TLR7 agonist and TLR8 agonist (TLR7/8L), as adjuvants, but was confirmed to significantly induce a Th1 immune response in a TLR2 agonist and a TRL3 agonist (TLR2/3L), for which a synergistic effect was previously confirmed. This immune response was confirmed to be unrelated to mouse species.

Additionally, the vaccine composition of the present disclosure showed effective inhibition of tumor growth in a tumor transplant model. These results may be seen as inhibiting the proliferation of transplanted cancer cells by inducing or maintaining a cytotoxic T cell response against transplanted cancer cells in the subject (mice) administered the vaccine composition of the present disclosure.

Therefore, the vaccine composition of the present disclosure may be used as an anticancer vaccine that may effectively inhibit tumor growth or recurrence.

Since the vaccine composition of the present disclosure aims to induce and/or maintain a therapeutic effect against cancer growth or recurrence, in the present disclosure, the vaccine composition of the present disclosure may be administered to a patient on any schedule suitable for inducing and/or maintaining a cytotoxic T cell response against HSP90. For example, a vaccine composition as described and exemplified herein may be administered to a patient on a schedule as a primary vaccination, followed by administrating a booster to induce and/or maintain immunity.

In some embodiments of the present disclosure, the vaccine composition may be administered to the patient once, twice, or more times per month. The booster may be administered in single or multiple doses at regular intervals, for example, at intervals of one month, after completion of the vaccination schedule, and may be administered in single or multiple doses over a period of 6 months or more. In some embodiments of the present disclosure, the vaccine composition may be administered three times, once a month, as a primary vaccination, and an additional booster may be administered after an arbitrary period of time, such as after 6 months or 1 year, etc.

The booster may have the same dose and composition as the vaccine composition used for primary vaccination, or the dosage and/or composition may be changed.

In addition to the HSP90 antigenic peptide and TLR2/3 agonist, the vaccine composition described herein may include a pharmaceutically acceptable excipient, carrier, diluent, buffer, stabilizer, preservative, or other substances well known in the art to which this disclosure belongs. However, it does not separately include a liposome. Such substances are non-toxic and do not interfere with the pharmaceutical activity of the active ingredient(s). The pharmaceutical carrier or diluent may be, for example, an aqueous solution. Carriers or other substances that may be included in the vaccine composition of the present disclosure may be selected differently depending on the route of administration (for example, oral, intravenous, dermal or subcutaneous, nasal, intramuscular, intradermal and intraperitoneal administration).

The vaccine compositions of the present disclosure may include one or more "pharmaceutically acceptable carriers". These typically include protein, saccharide, polylactic acid, polyglycolic acid, polymeric amino acid, amino acid copolymer, sucrose, and trehalose, etc. Such carriers are well known in the art to which this disclosure belongs. The vaccine composition may also contain a diluent, such as water, saline, glycerol, etc. In addition, auxiliary substance such as a lubricant or emulsifier, pH buffer substance, etc. may be present. Sterile pyrogen-free phosphate buffered saline (PBS), tromethamine (TRIS) are representative carriers.

The vaccine compositions of the present disclosure may additionally include an immunomodulator. Cytokines suitable for use as the immunomodulator may be selected from interleukin (for example, IL-1, IL-2, IL-4, IL-5, IL-6, IL-7, IL-12, etc.), macrophage colony stimulating factor (M-CSF), tumor necrosis factor (TNF), granulocyte macrophage colony stimulating factor (GM-CSF), etc., but is not limited thereto.

The vaccine composition of the present disclosure may be administered in combination with one or more anticancer agents selected from a chemical anticancer agent, a targeted anticancer agent, and an immune anticancer agent. One or more anticancer agents selected from the chemical anticancer agent, targeted anticancer agent, and immune anticancer agent that may be administered in combination with the vaccine composition of the present disclosure may be administered simultaneously or sequentially with a time difference, and may be selected according to an appropriate time and cycle.

In the present disclosure, chemical anticancer agent is also called a cytotoxic anticancer agent. The chemical anticancer agent include, for example, gemcitabine (gemcitabine injection), cytarabine, carboplatin (Paraplatin), cisplatin (Platinol, Platinol-AQ), crizotinib (Xalkori), cyclophosphamide (Cytoxan, Neosar), docetaxel (Taxotere), doxorubicin (Adriamycin), etoposide (Vepesid), fluorouracil (5-fluorouracil, 5-FU), irinotecan (Camptosar), liposome-encapsulated doxorubicin (doxil), methotrexate (Forlex, Maxate, Amethopterin), paclitaxel (Taxol, Abraxane), topotecan (Hycamtin), trabectedin (Yondelis), vincristine (Oncovin, Vincasar PFS) or vinbrastine (Velvan), capecitabine or oxaliplatin, etc., but is not limited thereto.

In the present disclosure, the targeted anticancer agent may be selected from a small molecule targeted anticancer agent, an antibody targeted anticancer agent, and an antibody-drug conjugate (ADC).

The small molecule targeted anticancer agent may be one or more selected from the group consisting of trametinib, vemurafenib, alpelisib, dactolisib, gefitinib, erlotinib, lapatinib, sunitinib, sorafenib, crizotinib, dabrafenib, trastuzumab, cetuximab, bevacizumab, panitumumab, ipilimumab, pertuzumab, tofacitinib, imatinib, and bortezomib, but is not limited thereto.

the antibody-targeted anticancer agent may be one or more selected from the group consisting of muromonab-CD3 abciximab, rituximab, daclizumab, palivizumab, infliximab, trastuzumab, etanercept, basiliximab, gemtuzumab, alemtuzumab, ibritumomab, adalimumab, alefacept, omalizumab, efalizumab, tositumomab, bevacizumab, natalizumab, ranibizumab, panitumumab, eculizumab, rilonacept, certolizumab, romiplostim, AMG-531, golimumab, ustekinumab, ABT-874, belatacept, belimumab, atacicept, anti-CD20 antibody, canakinumab, tocilizumab, atlizumab, mepolizumab, pertuzumab, HuMax CD20, tremelimumab, ticilimumab, ipilimumab, IDEC-114, inotuzumab, aflibercept, HuMax-CD4, teplizumab, otelixizumab, and catumaxomab, anti-EpCAM antibody IGN101, adecatumomab, oregovomab, dinutuximab, girentuximab, denosumab, and bapineuzumab, motavizumab, efumgumab, raxibacumab, LY2469298, and veltuzumab, but is not limited thereto.

The ADC may be one or more selected from the group consisting of gemtuzumab ozogamicin, brentuximab vedotin, and trastuzumab emtansine, Inotuzumab ozogamicin, polatuzumab vedotin, enfortumab vedotin, and trastuzumab deruxtecan, sacituzumab govitecan, belantamab mafodotin, moxetumomab pasudotox, loncastuximab tesirine, and tisotumab vedotin-tftv, but is not limited thereto.

In the present disclosure, the immune anticancer agent may be an immune checkpoint inhibitor against any one selected from the group consisting of cytotoxic T-lymphocyte-associated protein 4 (CTLA-4), programmed cell death protein 1 (PD-1), programmed death-ligand 1 (PD-L1), lymphocyte activation gene-3 (LAG-3), T-cell Immunoglobulin and mucin-domain containing-3 (TIM-3), T-cell Immunoreceptor with IG and ITIM domain (TIGIT), and V-domain Ig suppressor of T cell activation (VISTA), but is not limited thereto.

In the present disclosure, the composition may additionally include an anticancer adjuvant, and more preferably, the anticancer adjuvant may be a simulator of interferon gene (STING) agonist, but is not limited thereto.

The vaccine composition of the present disclosure may be used in a method of inducing an immune response in a subject or providing treatment, vaccination or immunotherapy, and the vaccine composition may be a vaccine or an immunotherapeutic composition.

In the present disclosure, the cancer may be a solid tumor.

In the present disclosure, the cancer may be characterized by one or more types selected from the group consisting of, for example, squamous cell cancer (for example, squamous cell cancer of the epithelium), small cell lung cancer, non-small cell lung cancer, lung cancer, peritoneal cancer, colon cancer, biliary tract tumor, nasopharyngeal cancer, laryngeal cancer, bronchial cancer, oral cancer, osteosarcoma, gallbladder cancer, kidney cancer, leukemia, bladder cancer, melanoma, brain cancer, glioma, brain tumor, skin cancer, pancreatic cancer, breast cancer, liver cancer, bone marrow cancer, esophageal cancer, colorectal cancer, stomach cancer, cervical cancer, prostate cancer, ovarian cancer, head and neck cancer, and rectal cancer, but is not limited thereto.

The vaccine compositions of the present disclosure may be lyophilized or in aqueous form. In the case of a lyophilized vaccine composition, all ingredients are reconstituted in an appropriate aqueous medium before administration to a patient. The aqueous form of the vaccine composition may be an aqueous solution or suspension. Liquid formulation of this type is ideal for injection as the composition may be administered directly in packaged form without the need for reconstitution in an aqueous medium. Vaccine compositions may be provided in a vial or provided as a prefilled syringe. The syringe may be supplied with or without a needle. The syringe may include a single dose, whereas the vial may include a single dose or multiple doses. In addition, the vaccine compositions of the present disclosure may be administered using a microneedle.

The vaccine compositions of the present disclosure may include an antimicrobial when packaged in a multiple dose format. The antimicrobial agent may be selected from 2-phenoxyethanol or parabens (methyl, ethyl, propyl paraben) etc. Any preservative is preferably present at a low level. The preservative may be added exogenously and/or may be an ingredient of the bulk antigen that is mixed to form the composition.

The vaccine composition of the present disclosure may be provided in the form of a kit including instructions, etc.

The term "therapeutically effective amount" used herein in combination with an active ingredient refers to an amount effective in preventing or treating the target disease, and the therapeutically effective amount of the composition of the present disclosure may vary depending on various factors, for example administration method, target region, patient condition, etc. Therefore, when used in the human body, the dosage should be determined as appropriate by considering both safety and efficiency. It is also possible to estimate the amount used in humans from the effective amount determined through animal testing.

Unless otherwise indicated, all numbers used in the specification and claims, whether mentioned or not, are to be understood in all cases may be modified by the term "about". Additionally, precise figures used in the specification and claims are to be understood as forming additional embodiments of the present disclosure. Efforts have been made to ensure the accuracy of the values disclosed in the examples. However, any measured value may inherently contain certain error value generated from the actual standard deviation in each measurement technique.

### Mode for Invention

Hereinafter, the present disclosure will be described in more detail through examples. These examples are solely for illustrating the present disclosure in more detail, and it will be apparent to those skilled in the art that the scope of the present disclosure is not limited by these examples according to the gist of the present disclosure.

### Examples

C57BL/6 mice (female, 7 weeks old), FVB mice (female, 6 weeks old), and MMTVneu transgenic mice (female, 6 weeks old) were selected as animals for measuring the immunogenicity of HSP90 antigenic peptides and anticancer effect according to the purpose of the experiment, and 4 animals were used in each group.

The animal tests in Example 1 were conducted ethically under the approval of the Institutional Animal Care and Use Committee (IACUC) of Cha University (approval number: #200081), and the animal tests in Examples 2 and 3 were conducted ethically under the approval of the Institutional Animal Care and Use Committee (IACUC) of Korea University (approval number: KOREA-2019-129).

The sequence information of the HSP90 antigenic peptide used in the experiment is listed in Table 1.

**[Table 1]**

| | Region of each peptide in the entire HSP90 sequence (Reference sequence: Gene ID: 3320) | Specific sequence |
|---|---|---|
| SEQ ID NO: 1 | p485-499 | LYVRRVFIMDNCEEL |
| SEQ ID NO: 2 | p527-541 | XSKILKVIRKNLVKK (X is pyroglutamate) |

### Example 1

To analyze the efficacy of antigenic peptides selected from HSP90 (see Table 1) and various adjuvants and liposomes, test vaccines were prepared with the compositions in Table 2 and Table 3. In the case of G6 and G7 formulations, since a liposome is included in the formulations, the drug release rate is slowed, and in this regard, the amount of antigenic peptide was reduced to 20% when preparing the test vaccine. The prepared test vaccine G1 to G7 formulations were immunized using C57BL/B6 mice (female, 7 weeks old), 4 mice per group. Freund's adjuvant (Complete Freund's adjuvant/Incomplete Freund's adjuvant) was used for CFA/IFA, Pam3Cys-SKKKK lipopolypeptide was used as a TLR2 agonist (TLR2L), and poly (I:C) was used as a TLR3 agonist (TLR3L). Cationic liposome was used as the liposome.

The test vaccine was prepared by mixing the antigenic peptide of the present disclosure with Pam3Cys-SKKKK (G3) or Poly (I:C) (G4) at room temperature, respectively, as described in previously filed registered patent No. 10-0900837 or No. 10-2098097, or using an L-pampo formulation (G5, G6, G7) including Pam3Cys-SKKKK and poly (I:C). The test vaccines of G3, G4, G5, G6, and G7 including TLR2 agonist and/or TLR3 agonist were prepared to include the contents of Table 2 or Table 3 in the total volume of 200 µl. In addition, the test vaccines G3, G4, G5, G6, and G7 were prepared in an aqueous solution formulation including 150 mM NaCl and 10 mM sodium phosphate, and the pH was set to 7.0.

The test vaccine was inoculated with each vaccine formulation by subcutaneous injection three times every 10 days for 4 mice in each group, and splenocytes were isolated from the spleens of mice sacrificed 10 days after the last inoculation, the specific response to each HSP90 antigenic peptide was measured through the ELISpot method for IFN-γ or IL-10 stimulated by the HSP90 antigenic peptide (stimulant) of SEQ ID NO: 1 or SEQ ID NO: 2 according to the previously known method by Disis et al. (J Clin Oncol. 2009 Oct 1; 27(28): 4685-92).

If HSP90 antigenic peptide-specific IFN-γ production increased in spleen cells measured by the ELISpot method after test vaccination, the Th1 immune response was considered activated, and if the HSP90 antigenic peptide-specific IL-10 production increased, the Th2 immune response was considered activated.

**[Table 2]**

| | G1 | G2 | G3 | G4 | G5 | G6 | G7 |
|---|---|---|---|---|---|---|---|
| HSP90 antigenic peptide (SEQ ID NO: 1) (µg) | - | 100 | 100 | 100 | 100 | 20 | 20 |
| CFA/IFA (µl) | - | 100 | - | - | - | - | - |
| TLR2L (µg) | - | - | 125 | - | 125 | 100 | 100 |
| TLR3L (µg) | - | - | - | 100 | 100 | 200 | 360 |
| liposome (µg) | - | - | - | - | - | 400 | 400 |

**[Table 3]**

| | G1 | G2 | G3 | G4 | G5 | G6 | G7 |
|---|---|---|---|---|---|---|---|
| HSP90 antigenic peptide (SEQ ID NO: 2) (µg) | - | 100 | 100 | 100 | 100 | 20 | 20 |
| CFA/IFA (µl) | - | 100 | - | - | - | - | - |
| TLR2L (µg) | - | - | 125 | - | 125 | 100 | 100 |
| TLR3L (µg) | - | - | - | 100 | 100 | 200 | 360 |
| liposome (µg) | - | - | - | - | - | 400 | 400 |

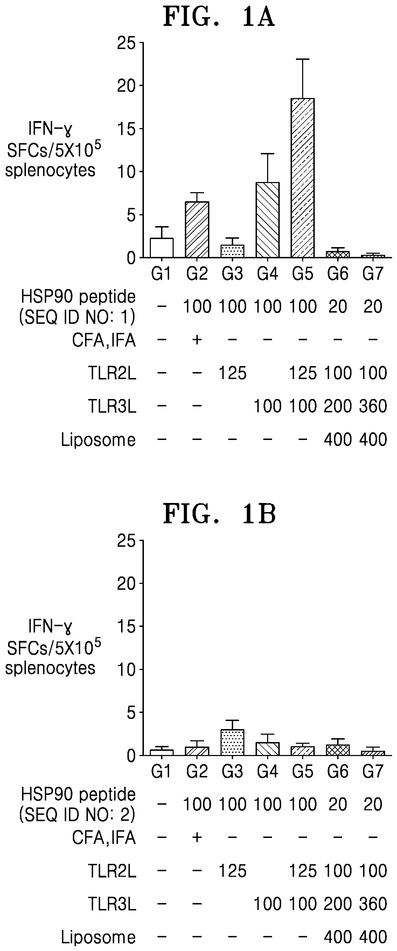

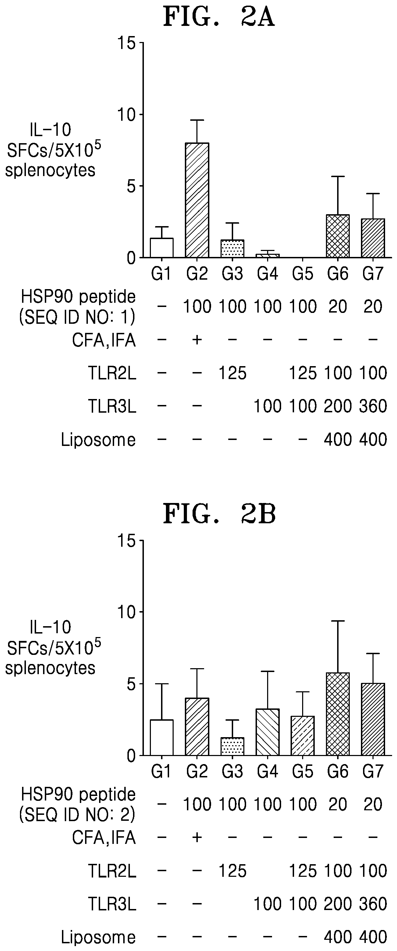

The ELISpot results for the G1 to G7 test vaccines including each antigenic peptide of SEQ ID Nos: 1 and 2 are shown in FIGS. 1 and 2.

In FIG. 1A, when the HSP90 antigenic peptide of SEQ ID NO: 1 was used as an antigen, the G3 formulation (including TLR2 agonist alone) showed little Th1 immune response, and the G4 formulation (including TLR3 agonist alone) showed an HSP90-specific Th1 immune response similar to that of the control group CFA/IFA. However, it was confirmed that the G5 formulation including both TLR2 agonist and TLR3 agonist showed a synergic effect compared to the G3 and G4 formulations, and when a liposome was added to the G5 formulation, the HSP90-specific Th1 immune response tended to rapidly decrease. In the case of the G7 formulation including a larger amount of TLR3 agonist, the HSP90-specific Th1 immune response tended to decrease more than that of the G6 formulation.

When the HSP90 antigenic peptide of SEQ ID NO: 2 was used as an antigen, almost no HSP90-specific Th1 immune response was observed regardless of the type of adjuvant and whether the liposome was included (FIG. 1B).

In FIG. 2A, when the HSP90 antigenic peptide of SEQ ID NO: 1 was used, the Th2 immune response was highest in the G2 formulation (CFA/IFA), and when the TLR2 agonist and/or TLR3 agonist were used, the Th2 immune response was hardly observed (G3-G5 formulation). However, when the liposome was included along with including the TLR2 agonist and the TLR3 agonist, there was a tendency for Th2 immunoreactivity to increase, but this was not statistically significant.

When using the HSP90 antigenic peptide of SEQ ID NO: 2, a Th2 immune response rather than a Th1 immune response may be observed, but no statistically significant results were obtained between each formulation (FIG. 2B).

Taken together, this experiment shows that the HSP90 antigenic peptide of SEQ ID NO: 1 effectively induces HSP90-specific T cell immune response, which is the effect required for a cancer vaccine, and that the mixed use of the TLR2 agonist and the TRL3 agonist as an adjuvant has a synergic effect (G5 formulation). However, it was confirmed that this synergistic effect tended to completely disappear when the liposome was added to the formulation. Therefore, it may be seen that the anticancer vaccine composition including the HSP90 antigenic peptide, which includes the TLR2 agonist and the TLR3 agonist as adjuvants and does not separately include the liposome, is a preferred formulation for HSP90-specific T cell immune response.

### Example 2

To analyze the activity of various TLR agonists as an adjuvant in a mouse model, a mixture of HSP90 antigenic peptides of SEQ ID NO:s 1 and 2 (hereinafter referred to as "HSP90 peptide") and a mixture of a TLR4 agonist (TLR4L), TLR2/3 agonist (TLR2 agonist and TRL3 agonist; TLR2/3L), TLR7/8 agonist (mixture of TLR7 agonist and TLR8 agonist; TLR7/8L) were formulated and FVB mice (female, 6 weeks old) were inoculated with each vaccine formulation by subcutaneous injection three times every 10 days, 4 mice per group, and 10 days after the last injection, splenocytes from the mice were isolated and HSP90-specific T cell responses were measured using the IFN-γ ELISpot method.

CFA/IFA used in this experiment was purchased from Sigma, and the TLR4 agonist used was GLA-SE from Quratis. TLR2/3 agonist was provided by Cha Vaccine Research Institute, and TLR7/8 agonist 3M-052 was purchased from 3M. The TT peptide used as a negative control group was a tetanus toxin peptide, and concanavalin A was used as a positive control group. As for the amount of each ingredient included in the vaccine formulation, 100 µg for CFA/IFA, 125 µg for TLR2 agonist, 100 µg for TLR3 agonist, and 100 µg for HSP90 peptide, a combination of 50 µg each of the peptide represented by SEQ ID NO: 1 and the peptide represented by SEQ ID NO: 2, and the remaining amount was adjusted to make a total volume of 200 µl with PBS.

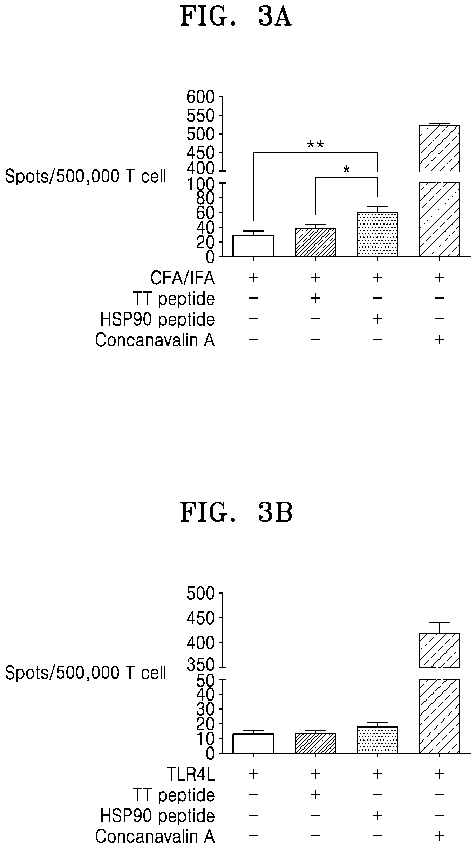

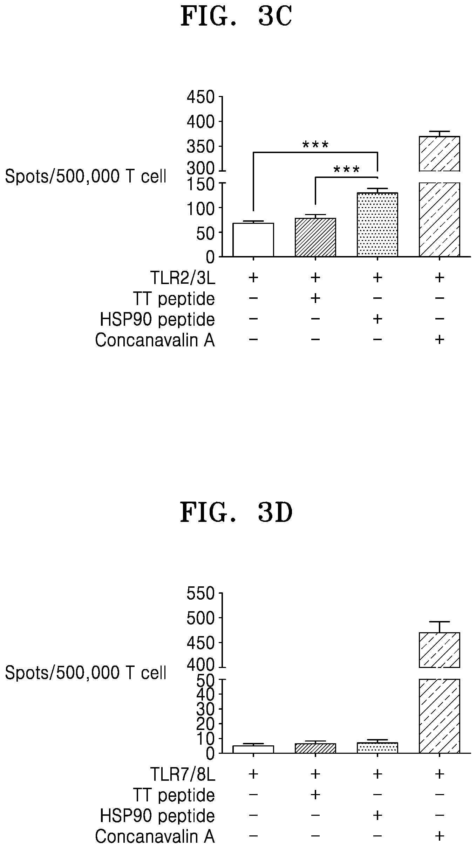

As shown in FIG. 3, none of the formulations including the HSP90 antigenic peptide and the TLR4 agonist or TLR7/8 agonist as an adjuvant failed to induce a HSP90-specific T cell response. Although the HSP90-specific T cell response was induced in both cases of using the CFAs/IFA and TLR2/3 agonist with HSP90 antigenic peptide and an adjuvant, when the TLR2/3 agonist was used, the HSP90-specific T cell response was induced twice or more higher than CFAs/IFA, which is generally known to induce a strong immune response in the anticancer vaccine. From this, it was confirmed that the TLR2/3 agonist is a more suitable adjuvant for the HSP90 antigenic peptide.

Therefore, when the HSP90 antigenic peptides of SEQ ID NOs: 1 and 2 were used as an immunogenic antigen, it was confirmed that among various TLR agonists, the TLR2/3 agonist specifically induced HSP90-specific T cell responses and these results reflect that when HSP90 antigenic peptide is used as an immunogen and the TLR2/3 agonist is used as an adjuvant, the desired immunogenic specific T cell response will be induced in the cancer vaccine.

### Example 3

A tumor transplant model used was a model in which mouse breast cancer cell lines were transplanted into mice. The mouse breast cancer cell line for tumor generation was a mouse mammary cancer cell-FVB/N-Tg (MMTVneu)-202Mul mouse-derived HER-2 overexpressing breast cancer cell line, and the mouse into which the breast cancer cell line was transplanted was a 6 weeks old female MMTVneu transgenic mouse.

Four animals per group were divided into a physiological saline (PBS) and adjuvant group (TLR2/3 agonist) group (hereinafter referred to as "control group") or an HSP90 antigenic peptide of SEQ ID NO: 1 and SEQ ID NO: 2 and an adjuvant group (TLR2/3 agonist) group (hereinafter referred to as "test group"). Mice were injected with the vaccine of the control group or the test group by subcutaneous injection three times every 10 days, and 10 days after the last injection, 5 × 10⁵ mouse breast cancer cell lines per mouse were injected subcutaneously into the flanks of mice in a 1:1 mixture of saline and matrigel, and the tumor growth was observed every 3-4 days to measure the size of the tumor to determine the effectiveness of the HSP90 antigenic peptide vaccine in inhibiting tumor formation. During the same period of vaccination and tumor cell transplantation, the body weight of each mouse did not differ between the control group and test group.

On day 35 after cancer cell transplantation, each mouse was sacrificed, cancer tissue and spleen were extracted, and the sizes were measured.

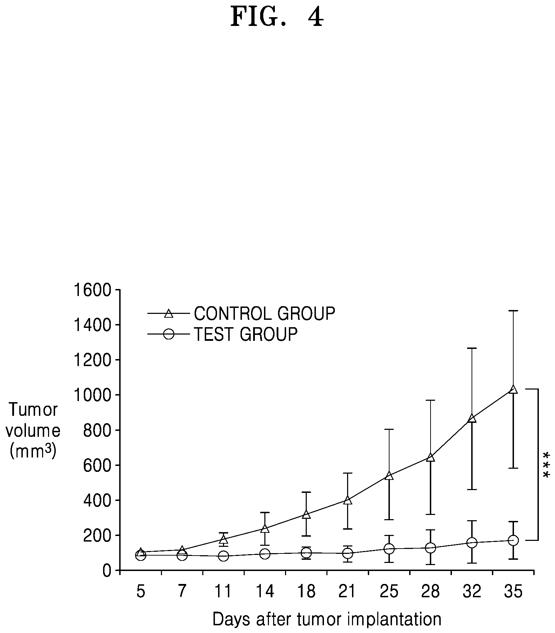

As shown in FIG. 4, the tumor growth of mice in the test group (HSP90 antigenic peptide and TLR2/3 agonist vaccine formulation administration group) was statistically significantly inhibited compared to the control group. Specifically, while the average tumor volume in control group was 1031 ± 450 mm³, the average tumor volume of test group was 170 ± 108 mm³, which was only 17% of the control group, confirming that the TLR2/3 agonist vaccine formulation may effectively inhibiting tumor formation in an antigen-specific tumor model. There was no difference in body weight between each group, confirming that the difference was not due to toxic reaction.

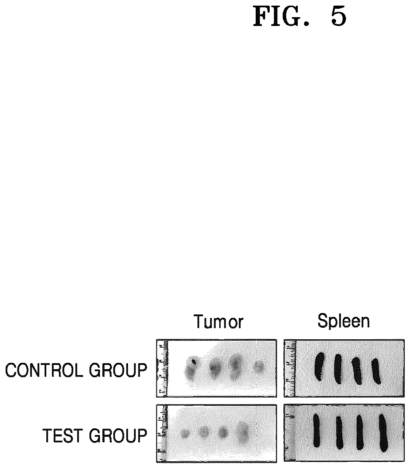

The sizes of the tumors and spleens extracted from the sacrificed control group and test group are shown in FIG. 5. The size of the spleen of the test group was clearly enlarged compared to the control group, and it was confirmed that the immune response was activated in the test group.

### Sequence List Text

1. SEQ ID NO: 1 (p485-499 region of the entire HSP90 sequence) LYVRRVFIMDNCEEL
2. SEQ ID NO: 2 (p527-541 region of the entire HSP90 sequence) XSKILKVIRKNLVKK (X is pyroglutamate)

## Claims

1. An anticancer vaccine composition comprising:
one or more peptides selected from the group consisting of a peptide comprising an amino acid sequence of SEQ ID NO: 1 and a peptide comprising an amino acid sequence of SEQ ID NO: 2; and
a TLR2 agonist and a TLR3 agonist as an adjuvant.

2. The anticancer vaccine composition of claim 1,
wherein the TLR2 agonist is one or more selected from Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, and Dhc-SKKKK.

3. The anticancer vaccine composition of claim 2,
wherein the TLR2 agonist is Pam3Cys-SKKKK.

4. The anticancer vaccine composition of claim 1,
wherein the TLR3 agonist is polyinosinic:polycytidylic acid (poly (I:C)).

5. The anticancer vaccine composition of claim 4,
wherein the poly (I:C) has a length of 50 bp to 2,000 bp.

6. An anticancer vaccine composition comprising:
one or more nucleic acids selected from the group consisting of a nucleic acid comprising an encoding sequence of a peptide comprising an amino acid sequence of SEQ ID NO: 1 and a nucleic acid comprising an encoding sequence of a peptide comprising an amino acid sequence of SEQ ID NO: 2; and
a TLR2 agonist and a TLR3 agonist as an adjuvant.

7. The anticancer vaccine composition of claim 6,
wherein the TLR2 agonist is one or more selected from Pam3Cys-SKKKK, PHC-SKKKK, Ole2PamCys-SKKKK, Pam2Cys-SKKKK, PamCys(Pam)-SKKKK, Ole2Cys-SKKKK, Myr2Cys-SKKKK, PamDhc-SKKKK, PamCSKKKK, and Dhc-SKKKK.

8. The anticancer vaccine composition of claim 7,
wherein the TLR2 agonist is Pam3Cys-SKKKK.

9. The anticancer vaccine composition of claim 6,
wherein the TLR3 agonist is polyinosinic:polycytidylic acid (poly (I:C)).

10. The anticancer vaccine composition of claim 9,
wherein the poly (I:C) has a length of 50 bp to 2,000 bp.

11. The anticancer vaccine composition of any one of claims 1 to 10,
wherein the anticancer vaccine composition is used to treat cancer and/or prevent cancer recurrence in a subject.

12. The anticancer vaccine composition of claim 11,
wherein the subject is a breast cancer patient.

13. The anticancer vaccine composition of any one of claims 1 to 10,
wherein the anticancer vaccine composition does not comprise a liposome.

14. The anticancer vaccine composition of any one of claims 1 to 10,
wherein the anticancer vaccine composition additionally comprises one or more agents selected from an immunomodulatory agent, a chemical anticancer agent, a targeted anticancer agent, and an immune anticancer agent.

15. The anticancer vaccine composition of claim 14,
wherein the immune anticancer agent is an immune checkpoint inhibitor against one or more selected from the group consisting of CTLA-4, PD-1, LAG-3, TIM-3, TIGIT, and VISTA.

16. A method of preventing, ameliorating, or treating cancer, the method comprising administering to a subject:
one or more peptides selected from the group consisting of a peptide comprising an amino acid sequence of SEQ ID NO: 1 and a peptide comprising an amino acid sequence of SEQ ID NO: 2; and
a TLR2 agonist and a TLR3 agonist, as an adjuvant.

17. Use of a peptide and an adjuvant for the manufacture of a medicament for prevention, amelioration, or treatment of cancer,
wherein the peptide is one or more selected from the group consisting of a peptide comprising an amino acid sequence of SEQ ID NO: 1 and a peptide comprising an amino acid sequence of SEQ ID NO: 2 and
a TLR2 agonist and a TLR3 agonist, as an adjuvant.
